(19) Europäisches Patentamt
European Patent Office
Office européen des brevets

(11) Veröffentlichungsnummer: **0 280 017**
**A1**

(12) # EUROPÄISCHE PATENTANMELDUNG

(21) Anmeldenummer: 88100441.0

(51) Int. Cl.[4]: **C07D 413/14**, A61K 31/42

(22) Anmeldetag: 14.01.88

Patentansprüche für folgende Vertragsstaaten: ES + GR.

(30) Priorität: **28.01.87 AT 164/87**

(43) Veröffentlichungstag der Anmeldung: **31.08.88 Patentblatt 88/35**

(84) Benannte Vertragsstaaten: **AT BE CH DE ES FR GB GR IT LI LU NL SE**

(71) Anmelder: **CL PHARMA AKTIENGESELLSCHAFT**
**St. Peter-Strasse 25**
**A-4021 Linz(AT)**

(72) Erfinder: **Binder, Dieter, Dr.**
**Sieveringerstrasse 207**
**A-1190 Wien(AT)**
Erfinder: **Rovenszky, Franz, Dipl.-Ing. Dr.**
**Lagerhausstrasse 5/8**
**A-2460 Bruck a.d. Leitha(AT)**
Erfinder: **Ferber, Hubert Peter, Dr.**
**Ahornweg 24**
**A-4021 Ansfelden(AT)**

(74) Vertreter: **Kunz, Ekkehard, Dr.**
**Chemie Holding AG Patentwesen St. Peter-Strasse 25**
**A-4021 Linz(AT)**

(54) **Neue Isoxazolderivate, Verfahren zu ihrer Herstellung und diese enthaltende pharmazeutische Präparate.**

(57) Die Erfindung betrifft neue substituierte Isoxazolderivate der allgemeinen Formel

in der $R_1$ $C_1$-$C_4$ Alkyl,
$R_2$ Wasserstoff, Chlor, Brom oder $C_1$-$C_4$ Alkyl,
$R_3$ und $R_4$ unabhängig voneinander Wasserstoff oder $C_1$-$C_4$ Alkyl, nicht aber beide Wasserstoff und
n eine ganze Zahl 6, oder 7 oder 8 bedeuten,
ein Verfahren zu ihrer Herstellung und pharmazeutische Präparate, die diese Verbindungen enthalten. Die neuen Verbindungen haben antivirale Wirkung und können als Wirkstoffe für Arzneimittel zur Behandlung und Prophylaxe von Viruserkrankungen verwendet werden.

EP 0 280 017 A1

# Neue Isoxazolderivate, Verfahren zu ihrer Herstellung und diese enthaltende pharmazeutische Präparate

Die Erfindung betrifft neue Isoxazolderivate, ein Verfahren für ihre Herstellung und ihre Verwendung in Medikamenten zur Bekämpfung viraler Infekte.

Aus der EP-A 211 157 sind bereits antiviral wirksame Isoxazolderivate bekannt, die jedoch in Position 4 des Oxazolringes unsubstituiert sind. Es wurde nun gefunden, daß eine Alkylierung des Oxazolringes in Position 4 die antivirale Wirksamkeit bedeutend zu steigern vermag.

Gegenstand der Erfindung sind daher neue Isoxazole der allgemeinen Formel I des Formelblattes

in der
$R_1$ $C_1$-$C_4$ Alkyl,
$R_2$ Wasserstoff, Chlor, Brom oder $C_1$-$C_4$ Alkyl,
$R_3$ und $R_4$ unabhängig voneinander Wasserstoff oder $C_1$-$C_4$ Alkyl, nicht aber beide Wasserstoff und
n eine ganze Zahl 6, 7 oder 8 bedeuten,

ein Verfahren zu ihrer Herstellung, pharmazeutische Präparate, die diese Verbindungen enthalten, sowie ihre Verwendung.

Die Verbindungen der allgemeinen Formel I können Asymmetriezentren enthalten. Die Erfindung betrifft auch die Racemate und Stereoisomeren der Verbindungen der allgemeinen Formel I.

Der in dieser Beschreibung verwendete Ausdruck "$C_1$-$C_4$ Alkyl" bezeichnet geradkettige oder verzweigte Kohlenwasserstoffgruppen mit 1 bis 4 Kohlenstoffatomen, wie z.B. Methyl, Ethyl, Propyl, Isopropyl, Butyl, tert. Butyl.

In einer bevorzugten Gruppe von Verbindungen der Formel I bedeutet $R_1$ Methyl oder Ethyl, wobei Methyl besonders bevorzugt ist. $R_2$ ist bevorzugt Wasserstoff und n steht bevorzugt für die Zahl 7. $R_3$ und $R_4$ bedeuten vorzugsweise Wasserstoff oder Methyl, wobei jedoch nicht beide Radikale Wasserstoff sein können.

Besonders bevorzugte Einzelverbindungen sind
5-(7-(5-(4,5-Dihydro-4,4-dimethyl-2-oxazolyl)-2-thienyl)oxyheptyl)-3-methyl-isoxazol und 5-(7-(5-(4,5-Dihydro-4-methyl-2-oxazolyl)-2-thienyl)oxyheptyl)-3-methyl-isoxazol.

Die Isoxazolderivate der allgemeinen Formel I können erfindungsgemäß dadurch hergestellt werden, daß man eine Verbindung der allgemeinen Formel II des Formelblattes, in der $R_1$, $R_2$, $R_3$, $R_4$ und n wie oben definiert sind, durch Behandeln mit einem wasserentziehenden Reagens unter Bildung des Oxazolrings cyclisiert.

Die erfindungsgemäße Umsetzung kann in An-oder Abwesenheit eines inerten organischen Lösungsmittels erfolgen.

Wird die Umsetzung in Anwesenheit eines Lösungsmittels durchgeführt, eignen sich als solche beispielsweise Kohlenwasserstoffe, wie Benzol, Toluol, Xylol, halogenierte Kohlenwasserstoffe, wie Chloroform, Chlorbenzol, Methylenchlorid, Tetrachlorkohlenstoff, Ether, wie Dioxan, Tetrahydrofuran, Dimethylformamid und dergleichen oder Mischungen solcher Lösungsmittel. Als wasserentziehende Mittel kommen dabei die für solche Cyclisierungen üblicherweise verwendeten Reagenzien in Frage, beispielsweise Phosphoroxychlorid, Phosphorpentachlorid, Thionylchlorid und dergleichen. Das wasserentziehende Reagens kann hiezu in äquivalenten Mengen oder in einem geringen Überschuß, beispielsweise in Mengen von 1,1 bis 3 Mol pro Mol der Verbindung der Formel II eingesetzt werden. Die Umsetzung erfolgt bei -20 °C bis +10 °C, vorzugsweise bei -5 °C bis +5 °C.

In einer besonders bevorzugten Ausführungsform des erfindungsgemäßen Verfahrens werden die Verbindungen der allgemeinen Formel II in Abwesenheit eines gesonderten Lösungsmittels durch Behandeln mit einem Überschuß eines flüssigen, wasserentziehenden Reagens, welches diesfalls zugleich als Lösungsmittel dient, bei -30° C bis +10° C, vorzugsweise -5° C bis + 5° C, ganz besonders bevorzugt im Eisbad bei etwa 0° C cyclisiert. Geeignete Reagenzien für diesen Zweck sind wiederum Phosphoroxychlorid oder Thionylchlorid, wobei sich die Verwendung von Thionylchlorid als ganz besonders vorteilhaft erwiesen hat.

Die für das erfindungsgemäße Verfahren verwendeten Ausgangsverbindungen der allgemeinen Formel II können, ausgehend von bekannten Produkten, in an sich bekannter Weise hergestellt werden. Insbesondere können sie, ausgehend von den in der EP-A 211 157 beschriebenen Verbindungen der Formel III gemäß dem folgenden Reaktionsschema und den spezifischen Angaben in den Beispielen synthetisiert werden:

Reaktionsschema

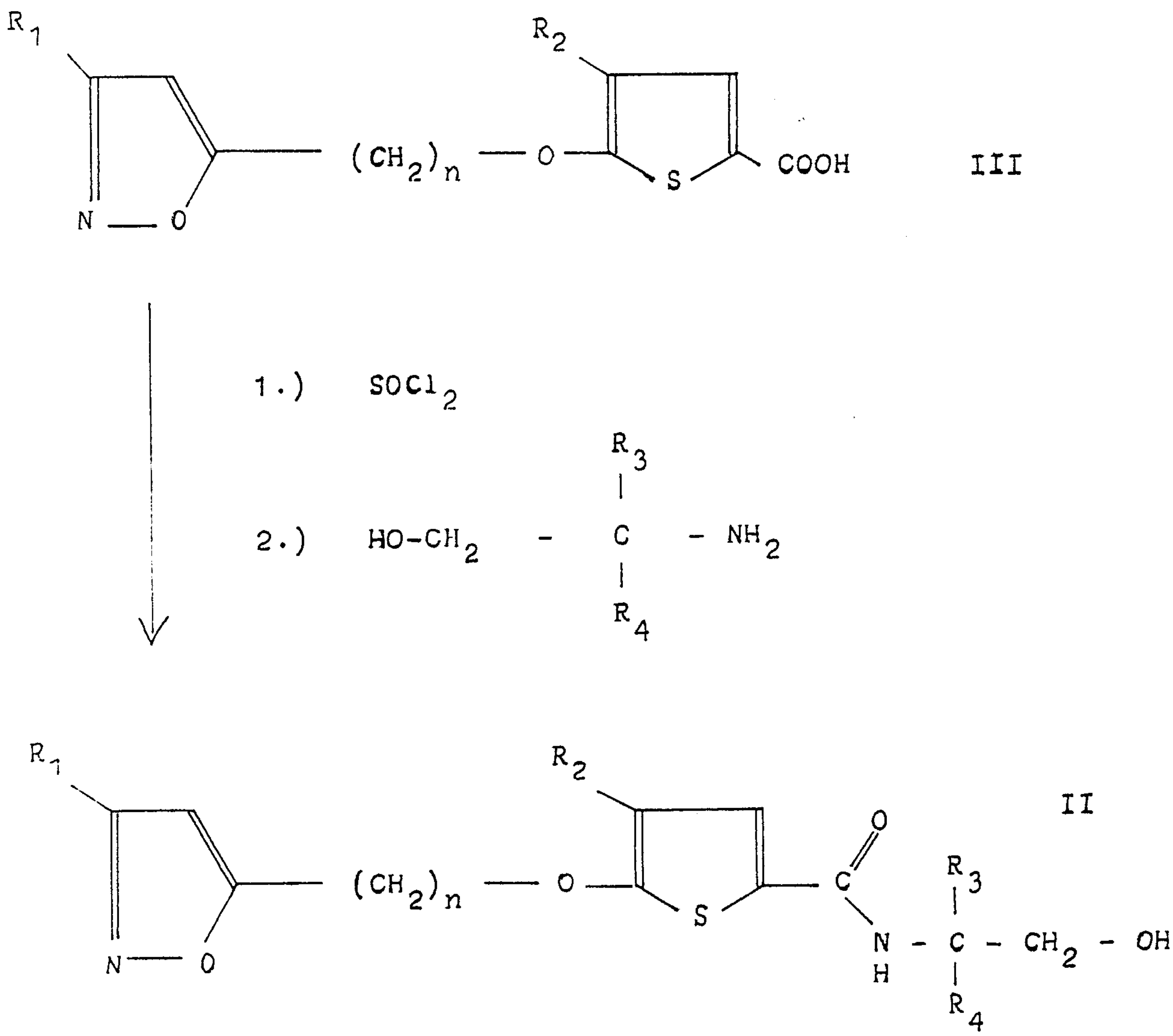


III

II

Die Verbindungen der allgemeinen Formel I haben eine antiinfektive, insbesondere eine ausgeprägte antivirale Wirkung. Diese wertvollen pharmakologischen Eigenschaften können in vitro und in vivo unter Verwendung von Standardmethoden bestimmt werden. Dabei zeigen die Verbindungen der allgemeinen Formel I insbesondere gegen verschiedene Typen von Retroviren, wie beispielsweise HIV-Viren und Picornaviren, eine hervorragende Wirkung und können daher in der Humanmedizin zur Behandlung und Prophylaxe von Viruserkrankungen eingesetzt werden. Zu den Picornaviren gehören unter anderem Rhino- und Enteroviren, zu denen beispielsweise Echo-, Coxackie-und Polioviren gerechnet werden.

Zur Untersuchung der antiviralen Eigenschaften wurde folgende Testmethode verwendet:

A: Allgemeiner antiviraler Test gegen verschiedene Viren:

In Mikrotiterplatten mit flachem Boden wurden serielle Dreifachverdünnungen von Lösungen der zu untersuchenden Substanzen in MEM (minimum essential medium) hergestellt. Gleiche Volumina der jeweiligen Virusverdünnungen in MEM und Zellsuspension in MEM mit 15 % FCS (fötales Kälberblutserum) wurden zugesetzt. Die Zellkonzentration wurde dabei so gewählt, daß sich nach 1 - 2 Tagen ein konfluenter Zellrasen bildet. Die Virusverdünnungen wurden so eingestellt, daß ohne Zusatz einer

Hemmsubstanz nach 3 - 4 Tagen ein vollständiger zytopathischer Effekt auftrat.
Als Kontrollen wurden Zellen (Zellkontrolle), Zellen mit Virus (Viruskontrolle) und Zellen mit den Testsubstanzen in verschiedener Konzentration (Toxizitätskontrolle) mitgeführt. Als minimale toxische Konzentration (MTK) wurde die Substanzkonzentration ermittelt, bei der eine noch geringere Zelldichte als in der Zellkontrolle beobachtet wurde.

Die Testsubstanzen wurden in Dimethylsulfoxid gelöst, in MEM verdünnt und mittels Ultraschall gut suspendiert.

Die erfindungsgemäßen Verbindungen wurden auf ihre antivirale Wirkung in einem repräsentativen Querschnitt an Retroviren untersucht und es wurde dabei die minimale Hemmkonzentration (MHK in µg/ml) ermittelt.

B: Test gegen HIV-Viren:

Der anti-HIV Test zur Bestimmung der anti-AIDS-Aktivität wurde nach Mitsuya, H. et al, "Rapid in vitro systems for assessing activity of agents against HTLV-III/LAV", in: AIDS: Modern Concepts and Chemotherapeutic Challenges (S. Broder, ed) Marcel Dekker, Inc., New York, 303 - 333 (1986) durchgeführt:

Menschliche T-Lymphozyten - ATH8-Zellen wurden mit 2 mg/ml Polybrene für 30 Minuten bei 37 °C vorbehandelt. Die Zellen wurden dann abzentrifugiert, in frischem RPMI - 1640 Kulturmedium, das 13 % fötales Kälberserum, 11 % Interleukin-2 (v/v), 50 µM beta-Mercaptoäthanol, 4 mM L-Glutamin, 50 E/ml Penicillin und 50 mg/ml Streptomycin enthielt, resuspendiert und mit 2 × $10^3$ Virions/Zelle für 60 - 90 Minuten bei 37 °C infiziert. (Die HTLV-$III_B$-Viren wurden von einem Pool amerikanischer AIDS-Patienten erhalten. Ungefähr $6.10^{10}$ Viruspartikel/ml wurden aus dem Überstand von HIV-produzierenden H9-Zellkulturen erhalten, wie bei Mitsuya, H. et al. Proc. Natl. Acad. Sci. USA 82, 7096 - 7100 (1985) beschrieben.

Diese Viruskonzentration stellt die 400-fache Minimaldosis dar, die zur Induzierung der Zellpathogenität in ATH8-Zellen notwendig ist und führt daher zu einer sehr hohen Multiplizität der Infektion. Nach der Infizierung wurden die Zellen ins Kulturmedium rückgeführt, gemischt und je 2 ml in An-oder Abwesenheit der Testsubstanzen in Kulturflaschen überführt. Nach Inkubation bei 37° C für 6 bis 7 bzw. 10 Tage wurde die Zahl der lebenden Zellen gezählt und mit der Kontrolle ohne Testsubstanz verglichen.

In beiden Testsystemen zeigen die Verbindungen der vorliegenden Anmeldung eine mehrfach bessere Wirkung als die Verbindungen der EP-A 211 157.

Die Verbindungen der allgemeinen Formel I können auf übliche Weise, wie beispielsweise oral oder parenteral, verabreicht werden. Vorzugsweise werden sie oral verabreicht, wobei die Tagesdosis ca. 0.005 bis 10 mg/kg Körpergewicht beträgt, vorzugsweise 0,05 bis 0,5 mg/kg Körpergewicht. Der behandelnde Arzt kann jedoch, abhängig vom Allgemeinzustand des Patienten, der entsprechenden Substanz der Formel I und der Art der Formulierung, auch Dosen darüber oder darunter verschreiben.

Falls die erfindungsgemäßen Substanzen zur Prophylaxe von Viruserkrankungen verwendet werden, bewegen sich die Dosen ungefähr im selben Rahmen wie im Behandlungsfall. Die orale Verabreichung ist auch im Fall der Prophylaxe bevorzugt.

Die Verbindungen der Formel I können allein oder in Verbindung mit anderen pharmazeutisch aktiven Substnazen verabreicht werden, wobei der Gehalt der Verbindungen der Formel I zwischen 0,1 und 99 % liegt. Im allgemeinen liegen die pharmazeutisch aktiven Verbindungen in einer Mischung mit geeigneten inerten Hilfs-, Träger-und/oder Verdünnungsmitteln, wie z.B. pharmazeutisch unbedenklichen Lösungsmitteln, Gelatine, Gummi arabicum, Milchzucker, Stärke, Magnesiumstearat, Talk, pflanzlichen Ölen, Polyalkylenglykol, Vaseline und dergleichen vor.
Die pharmazeutischen Präparate können in fester Form, beispielsweise als Tabletten, Dragees, Suppositorien, Kapseln und dergleichen, in halbfester Form, beispielsweise als Salben oder in flüssiger Form, beispielsweise als Lösungen, Suspensionen oder Emulsionen, vorliegen. Gegebenenfalls sind sie sterilisiert und enthalten Hilfsstoffe, wie Konservierungs-, Stabilisierungs-oder Emulgiermittel, Salze zur Veränderung des osmotischen Druckes und dergleichen.
Insbesondere können pharmazeutische Präparate die erfindungsgemäßen Verbindungen in Kombination mit anderen therapeutisch wertvollen Stoffen, beispielsweise mit anderen antiinfektiven oder antiviralen Wirkstoffen, enthalten. Mit diesen können die erfindungsgemäßen Verbindungen beispielsweise zusammen mit den oben angegegenen Hilfs-, Träger-und/oder Verdünnungsmitteln zu Kombinationspräparaten formuliert werden.

Beispiel 1:

5-(7-(5-(4,5-Dihydro-4,4-dimethyl-2-oxazolyl)-2-thienyl)oxyheptyl)-3-methylisoxazol

0,85 g (2,2 mmol) N-(2-Hydroxy-1,1-dimethyl-ethyl)-5-(7-(3-methyl-5-isoxazolyl)heptyloxy)-2-thiophencarbonsäureamid werden in 5 ml Thionylchlorid bei Raumtemperatur eingetragen und noch 20 min. gerührt. Danach wird vorsichtig eingedampft und der Rückstand zwischen 20 ml gesättigter Natriumcarbonatlösung und 30 ml Ethylacetat verteilt. Die wäßrige Phase wird noch zweimal mit je 30 ml Ethylacetat extrahiert. Die vereinigten organischen Phasen werden über Natrimsulfat getrocknet, filtriert und eingedampft.
Ausbeute: 0,7 g bräunliches Öl
Das Rohprodukt wird säulenchromatographisch gereinigt (Kieselgel 60, Eluens: Ethylacetat Petrolether = 1:1).
Ausbeute: 320 mg farbloses Öl, das im Tiefkühlschrank kristallisiert. (39,5 % der Theorie).
Schmelzpunkt: liegt unter Raumtemperatur
$^1$H-NMR ($CDCl_3$: delta (ppm) = 7,28; 7,24; 6,18; 6,13 (AB, 2H, Th-$H_3$, Th-$H_4$); 5,80 (s, 1H, Isox-$H_4$); 4,05 (t, 2H, O-$CH_2$); 4,05 (s, 2H, Oxaz-$CH_2$; 2,70 (t, 2H, Isox-$CH_2$); 2,25 (s, 3H, Isox-$CH_3$); 1,95-1,16 (m, 1OH, $CH_2$); 1,10 (s, 6H, 2 $CH_3$).

Das Ausgangsmaterial kann wie folgt hergestellt werden:

N-(2-Hydroxy-1,1-dimethyl-ethyl)-5-(7-(3-methyl-5-isoxazolyl)heptyloxy)-2-thiophencarbonsäureamid

1,0 g (3,1 mmol) 5-(7-(3-Methyl-5-isxazolyl)heptyloxy)-2-thiophencarbonsäure werden in 10 ml Thionylchlorid bei 0°C eingerührt. Es wird noch 20 min. bei dieser Temperatur gerührt und überschüssiges Thionylchlorid bei einer Badtemperatur von 30° C im Vakuum abdestilliert. Der Rückstand wird von Thionylchlorid-Resten im Vakuum befreit und in 7 ml abs. Methylenchlorid gelöst. Diese Lösung wird unter Rühren bei einer Temperatur zwischen 0 und 5 °C zu einer Lösung von 0,61 g (6,8 mmol) 2-Amino-2-methyl-1-propanol in 7 ml abs. Methylenchlorid innerhalb von 40 min. unter Rühren zugetropft. Es wird auf Raumtemperatur erwärmen gelassen und noch 1 Stunde gerührt. Anschließend wird das Reaktionsgemisch auf 10 ml 2n HCl gegossen, die Phasen getrennt und die wäßrige noch zweimal mit je 30 ml Methylenchlorid extrahiert. Die vereinigten organischen Phasen werden mit 10 ml Wasser gewaschen, über Natriumsulfat getrocknet, filtriert und eingedampft.
Ausbeute: 1,1 g bräunliche Kristalle (90,2 % d.Th.)
Schmelzpunkt: 73 - 75 °C (Acetoniril)

Beispiel 2:

R,S-5-(7-(5-(4,5-Dihydro-4-methyl-2-oxazolyl)-2-thienyl)oxypheptyl)-3-methylisoxazol

0,42 g (1,1 mmol) R,S-N-(2-Hydroxy-1-methyl-ethyl)-5-(7-(3-methyl-5-is-o xazolyl)heptyloxy)-2-thiophencarbonsäureamid werden in 5 ml Thionylchlorid bei Raumtemperatur eingetragen und noch 20 min. gerührt. Danach wird vorsichtig eingedampft und der Rückstand zwischen 20 ml ges. Natriumbicarbonatlösung und 30 ml Ethylacetat verteilt. Die wäßrige Phase wird noch zweimal mit je 30 ml Ethylacetat extrahiert. Die vereinigten organischen Phasen werden über Natriumsulfat getrocknet, filtriert und eingedampft.
Ausbeute: 0,39 g bräunliches Öl (97,5 % d.Th.)
Das Rohprodukt wird säulenchromatographisch gereinigt (Kieselgel 60, Eluens: Ethylacetat/Petrolether = 1:1).
Ausbeute: 120 mg farblose Kristalle (30,0 % d. Th.
Schmelzpunkt: 43,5 - 45 °C (Diisopropylether)

$^1$H-NMR: ($CDCl_3$): delta (ppm) = 7,26; 7,23; 6,18; 6,13 (AB, 2H, Th-$H_3$, Th-$H_4$); 5,80 (s, 1H, Isox-$H_4$); 4,48-3,82 (m, 5H, O-$CH_2$-CH-N u. O-$CH_2$); 2,70 (t, 2H, Isox-$CH_2$); 2,26 (s, 3H, Isox-$CH_3$); 1,91 - 1,17 (m, 1OH, $CH_2$); 1,34 (d; 3H, -$CH_3$).

Das Ausgangsmaterial kann wie folgt hergestellt werden:

R,S-N-(2-Hydroxy-1-methyl-ethyl)-5-(7-(3-methyl-5-isoxazolyl)heptyloxy)-2-thiophencarbonsäureamid

0,5 g (1,5 mmol) 5-(7-(3-Methyl-5-isoxazolyl)heptyloxy)-2-thiophencarbonsäure werden in 6 ml Thionylchlorid bei 0 °C eingerührt. Es wird noch 20 min. bei dieser Temperatur gerührt und überschüssiges Thionylchlorid bei einer Badtemperatur von 30 °C im Vakuum abdestilliert. Der Rückstand wird von Thionylchorid-Resten im Vakuum befreit und in 4 ml absolutem Methylenchlordid gelöst. Diese Lösung wird unter Rühren bei einer Temperatur zwischen -10 und -15 °C zu einer Lösung von 0.26 g (3.4 mmol) R.S-2-Amino-1-propanol in 4 ml absolutem Methylenchlorid innerhalb von 40 min. unter Rühren zugetropft. Es wird auf Raumtemperatur erwärmen gelassen und noch 1 Stunde gerührt. Anschließend wird das Reaktionsgemisch auf 10 ml 2n HCl gegossen, die Phasen getrennt und die wäßrige noch zweimal mit je 30 ml Methylenchlorid extrahiert. Die vereinigten organischen Phasen werden mit 10 ml Wasser gewaschen. über Natriumsulfat getrocknet, filtriert und eingedampft.
Ausbeute: 0,49 g bräunliche Kristalle (83,3 % d.Th.)
Schmelzpunkt: 84 - 86 °C (Acetonitril)

Beispiel 3:

S-5-(7-(5-(4,5-Dihydro-4-methyl-2-oxazolyl)-2-thienyl)oxyheptyl)-3-methylisoxazol

1,1 g (2,9 mmol) S-N-(2-Hydroxy-1-methyl-ethyl)-5-(7-(3-methyl-5-isoxazolyl)-heptyloxy)-2-thiophencarbonsäureamid werden in 15 ml Thionylchlorid bei Raumtemperatur eingetragen und noch 20 min. gerührt. Danach wird vorsichtig eingedampft und der Rückstand zwischen 20 ml ges. Natriumbicarbonatlösung und 30 ml Ethylacetat verteilt. Die wäßrige Phase wird noch zweimal mit je 30 ml Ethylacetat extrahiert. Die vereinigten organischen Phasen werden über Natriumsulfat getrocknet, filtriert und eingedampft.
Ausbeute: 0,96 g bräunliche Kristalle (91,6 % d.Th.)
Das Rohprodukt wird aus Diisopropylether umkristallisiert.
Ausbeute: 0,96 g (91,6 % d. Th.)
Schmelzpunkt: 53 - 55 °C (Diisopropylether)

$^1$H-NMR ($CDCl_3$): delta (ppm) = 7,28; 7.23; 6,18; 6,13 (AB, 2H, Th-$H_3$, Th-$H_4$); 5,79 (s, 1H, Isox-$H_4$); 4.46-3,87 (m, 5H, O-$CH_2$-CH-N u.O-$CH_2$); 2.70 (t, 2H, Isox-$CH_2$); 2,25 (s, 3H, Isox-$CH_3$); 1,95 - 1,16 (m, 10H, $CH_2$); 1,34 (d, 3H, -$CH_3$).

Das Ausgangsmaterial kann wie folgt herstellt werden:

S-N-(2-Hydroxy-1-methyl-ethyl)-5-(7-(3-methyl-5-isoxazolyl)heptyloxy)-2-thiophencarbonsäureamid

1,1 g (3,4 mmol) 5-(7-(3-Methyl-5-isoxazolyl)heptyloxy)-2-thiophencarbonsäure werden in 10 ml Thionylchlorid bei 0 °C eingerührt. Es wird noch 20 min. bei dieser Temperatur gerührt und überschüssiges Thionylchlorid bei einer Badtemperatur von 30 °C im Vakuum abdestilliert. Der Rückstand wird von Thionylchlorid-Resten im Vakuum befreit und in 10 ml absolutem Methylenchlorid gelöst. Diese Lösung wird unter Rühren bei einer Temperatur zwischen -10 und -15 °C zu einer Lösung von 0,57 g (7.6 mmol) S-2-Amino-1-propanol in 10 ml abs. Methylenchlorid innerhalb von 40 min. unter Rühren zugetropft. Es wird auf Raumtemperatur erwärmen gelassen und noch 1 Stunde gerührt. Anschließend wird das Reaktionsgemisch auf 20 ml 2n HCl gegossen, die Phasen getrennt und die wäßrige noch zweimal mit je 50 ml Methylenchlorid extrahiert. Die vereinigten organischen Phasen werden mit 10 ml Wasser gewaschen, über Natriumsulfat getrocknet, filtriert und eingedampft.
Ausbeute: 1,2 g bräunliche Kristalle (93,0 % d.Th.)
Schmelzpunkt: 82 - 85 °C (Acetonitril)

Beispiel 4:

5-(7-(5-(4.5-Dihydro-4,4-dimethyl-2-oxazolyl)-2-thienyl)oxyheptyl)-3-methylisoxazol

1,0 g (2,5 mmol) N-(2-Hydroxy-1,1-dimethyl-ethyl)-5-(7-(3-methyl-5-isoxazolyl)-heptyloxy)-2-thiophencarbonsäureamid werden in 5 ml Phosphoroxychlorid bei -10 °C eingetragen und noch 10 Minuten gerührt. Danach wird im Vakuum vorsichtig eingedampft und der Rückstand zwischen 25 ml ges. Natriumbicarbonatlösung und 40 ml Ethylacetat verteilt. Die wäßrige Phase wird noch zweimal mit je 20 ml Ethylacetat extrahiert. Die vereinigten organischen Phasen werden über Natriumsulfat getrocknet, filtriert und eingedampft. Das Rohprodukt wird säulenchromatographisch gereinigt, wobei das gewünschte Produkt als erstes eluiert wird (Kieselgel 60, Eluens: Ethylacetat/Petrolether = 1:1). Ausbeute: 0.21 g farbloses Öl (22.1 % d. Th.), das im Tiefkühlschrank kristallisiert.
Schmelzpunkt: liegt unter Raumtemperatur

$^1$H-NMR: ($CDCl_3$: delta (ppm) = 7,28; 7,24; 6,18; 6,13; (AB, 2H, Th-$H_3$, Th-$H_4$); 5,80 (s, 1H, Isox-$H_4$); 4,05 (t, 2H, O-$CH_2$); 4,05 (s, 2H, Oxaz-$CH_2$; 2,70 (t, 2H, Isox-$CH_2$); 2,25 (s, 3H, Isox-$CH_3$); 1,95-1,16 (m, 10H, $CH_2$); 1,10 (s, 6H, 2 $CH_3$).

## Ansprüche

1. Substituierte Isoxazolderivate der allgemeinen Formel I des Formelblattes

in der
R. $C_1$-$C_4$ Alkyl,
$R_2$ Wasserstoff, Chlor, Brom oder $C_1$-$C_4$ Alkyl,
$R_3$ und $R_4$ unabhängig voneinander Wasserstoff oder $C_1$-$C_4$ Alkyl, nicht aber beide Wasserstoff und
n eine ganze Zahl 6, 7 oder 8 bedeuten.

2. Verbindungen der allgemeinen Formel I nach Anspruch 1, worin $R_1$ Methyl, $R_2$ Wasserstoff und n eine ganze Zahl 7 bedeuten.

3. Verbindungen der allgemeinen Formel I nach einem der Ansprüche 1 oder 2, worin $R_3$ und $R_4$ unabhängig voneinander Wasserstoff oder Methyl bedeuten, nicht jedoch beide Wasserstoff.

4. Die Verbindung nach Anspruch 1, 5-(7-(5-(4,5-Dihydro-4,4-dimethyl-2-oxazolyl)-2-thienyl)oxyheptyl)-3-methyl-isoxaxol.

5. Die Verbindung nach Anspruch 1, 5-(7-(5-(4,5-Dihydro-4-methyl-2-oxazolyl)-2-thienyl)oxyheptyl)-3-methyl-isoxaxol.

6. Verfahren zur Herstellung von Verbindungen der im Anspruch 1 definierten allgemeinen Formel I, dadurch gekennzeichnet, daß man eine Verbindung der allgemeinen Formel II des Formelblattes

in der
$R_1$, $R_2$, $R_3$, $R_4$ und n wie in oben definiert sind,

durch Behandeln mit einem wasserentziehenden Reagens unter Bildung des Oxazolrings cyclisiert.

7. Pharmazeutische Präparate, enthaltend Verbindungen der allgemeinen Formel I nach Anspruch 1 in Kombination mit üblichen galenischen Hilfs-, Träger-und/oder Verdünnungsmitteln.

8. Pharmazeutische Präparate, enthalten Verbindungen der allgemeinen Formel I nach Anspruch 1 in Kombination mit anderen antiinfektiven oder antiviralen Wirkstoffen sowie üblichen galenischen Hilfs-, Träger-und/oder Verdünngungsmitteln.

9. Verbindungen nach Anspruch 1 zur Verwendung als Wirkstoffe für Arzneimittel zur Behandlung und Prophylaxe von Viruserkrankungen.

Patentansprüche für folgenden Vertragsstaaten : GR und ES

1. Verfahren zur Herstellung neuer substituierter Isoxazolderivate der allgemeinen Formel I des Formelblattes,
in der
R. $C_1$-$C_4$ Alkyl,
$R_2$ Wasserstoff, Chlor, Brom oder $C_1$-$C_4$ Alkyl,
$R_3$ und $R_4$ unabhängig voneinander Wasserstoff oder $C_1$-$C_4$ Alkyl, nicht aber beide Wasserstoff und
n eine ganze Zahl 6, 7 oder 8 bedeuten,

dadurch gekennzeichnet,
daß man eine Verbindung der allgemeinen Formel II des Formelblattes,
in der
R , $R_2$, $R_3$, $R_4$ und n wie oben definiert sind,
durch Behandeln mit einem wasserentziehenden Reagens unter Bildung des Oxazolrings cyclisiert.

2. Verfahren nach Anspruch 1, dadurch gekennzeichnet, daß man eine Verbindung der allgemeinen Formel II, worin R Methyl, $R_2$ Wasserstoff und n eine ganze Zahl 7 bedeuten, cyclisiert.

3. Verfahren nach einem der Ansprüche 1 oder 2, dadurch gekennzeichnet, daß man eine Verbindung der allgemeinen Formel II, worin $R_3$ und $R_4$ unabhängig voneinander Wasserstoff oder Methyl bedeuten, nicht jedoch beide Wasserstoff, cyclisiert.

4. Verfahren nach Anspruch 1, dadurch gekennzeichnet, daß man eine Verbindung der allgemeinen Formel II, worin R Methyl, $R_2$ Wasserstoff, $R_3$ und $R_4$ Methyl und n die Zahl 7 bedeuten, cyclisiert.

5. Verfahren nach Anspruch 1, dadurch gekennzeichnet, daß man eine Verbindung der allgemeinen Formel II, worin R Methyl, $R_2$ und $R_3$ Wasserstoff, $R_4$ Methyl und n die Zahl 7 bedeuten, cyclisiert.

6. Verfahren nach einem der Ansprüche 1 bis 5, dadurch gekennzeichnet, daß man die Cyclisierung in einem inerten Lösungsmittel in Gegenwart von Phosphoroxychlorid, Phosphorpentachlorid oder Thionylchlorid bei -5 °C bis +5 °C durchführt.

7. Verfahren nach Anspruch 6, dadurch gekennzeichnet, daß man das wasserentziehende Reagens in einem 1.1 bis 3-fachen molaren Überschuß pro Mol der Verbindung der Formel II einsetzt.

8. Verfahren nach einem der Ansprüche 1 bis 5, dadurch gekennzeichnet, daß man die Cyclisierung in einem Überschuß eines flüssigen waserentziehenden Reagens ohne gesondertes Lösungsmittel bei -15 bis +5 °C durchführt.

9. Verfahren nach Anspruch 8, dadurch gekennzeichnet, daß man zur Cyclisierung Thionylchlorid verwendet.

Formelblatt

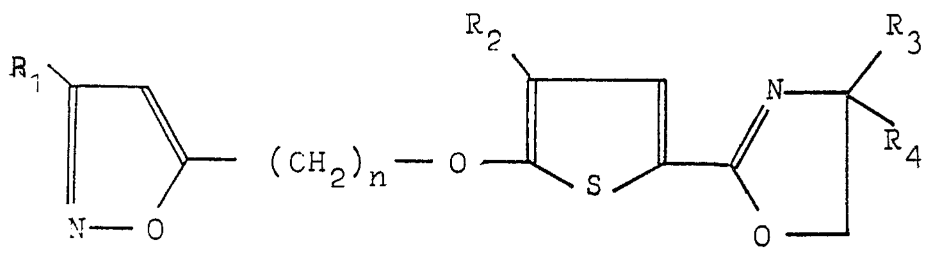

I

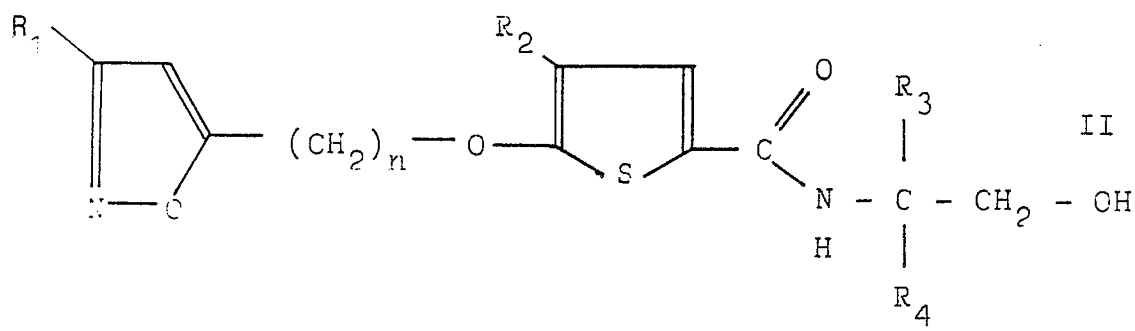

II

Europäisches Patentamt

# EUROPÄISCHER RECHERCHENBERICHT

Nummer der Anmeldung

EP 88 10 0441

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| --- | --- | --- | --- |
| A | EP-A-0 137 242 (STERLING DRUG)<br>* Ansprüche 1,10 *<br>--- | 1,7-9 | C 07 D 413/14<br>A 61 K 31/42 |
| A | EP-A-0 111 345 (STERLING DRUG)<br>* Ansprüche 1,9 *<br>--- | 1,7-9 | |
| D,P<br>X | EP-A-0 211 157 (CHEMIE LINZ)<br>* Ansprüche *<br>----- | 1,6-9 | |
| | | | **RECHERCHIERTE SACHGEBIETE (Int. Cl.4)** |
| | | | C 07 D 413/00 |

Der vorliegende Recherchenbericht wurde für alle Patentansprüche erstellt

| Recherchenort | Abschlußdatum der Recherche | Prüfer |
| --- | --- | --- |
| DEN HAAG | 15-04-1988 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)